# EUROPEAN PATENT APPLICATION

(11) **EP 2 561 809 A1**
(43) Date of publication of application: **27.02.2013**
(21) Application number: 12155534.6
(22) Date of filing: 15.02.2012
(51) Int. Cl.: A61B 5/055, A61B 5/103, A61B 6/04

(54) **Magnetic resonance imaging (MRI) system and method of controlling the same**

(30) Priority: 22.08.2011 KR 20110083574
(71) Applicant: Samsung Electronics Co., Ltd., Suwon-si, Gyeonggi-do, 443-742 (KR)
(72) Inventor: Hwang, Jin-young, Suwon-si Gyeonggi-do (KR); Han, Woo-sup, Yongin-si Gyeonggi-do (KR); Kwon, Oh-soo, Seoul (KR)
(74) Representative: Grünecker, Kinkeldey, Stockmair & Schwanhäusser

(57) **Abstract**

A magnetic resonance imaging (MRI) system obtains a magnetic resonance image of a patient by obtaining a body profile of the patient, transferring the patient inside a magnet of the MRI system based on the obtained body profile, and using a magnetic field of an RF coil based on the obtained body profile.

## Description

### CLAIM OF PRIORITY

This application claims the benefit under 35 U.S.C. § 119(a) of the earlier filing date of Korean Patent Application No. 10-2011-0083574. filed on August 22, 2011, in the Korean Intellectual Property Office, the entire disclosure of which is incorporated herein in by reference.

### BACKGROUND OF THE INVENTION

### 1. Field of the Invention

The present invention relates to a magnetic resonance imaging (MRI) system and a method of controlling the same.

### 2. Description of the Related Art

MRI systems are used to obtain tomographic images of biological tissues in the human body by using a magnetic field generated by magnetic force. Among such MRI systems, in particular, an MRI device obtains tomographic images of biological tissues by using a resonance phenomenon whereby a magnetic field is applied to the body of a patient who lies on a table device that has been transferred into the MRI device. Thus, the table device is used as a member for transferring a patient inside or outside the MRI device.

### SUMMARY OF THE INVENTION

The present invention provides a MRI system for obtaining a body profile of a patient.

The present invention also provides a method of controlling the MRI system by obtaining a body profile of a patient and using the body profile of the patient. The present invention is not limited to these technical goals and other technical goals may be inferred from the following exemplary embodiments.

According to another aspect of the present invention, there is provided a method of controlling an MRI system, the method including: obtaining a body profile of a patient; transferring the patient to a predetermined position inside a magnet of the MRI system based on the obtained body profile; and obtaining a magnetic resonance image of the patient by using a magnetic field of an RF coil generated by a parameter of a pulse sequence based on the obtained body profile. Furthermore, the transferring step may sequentially transfer the patient to a plurality of predetermined positions inside the magnet of the MRI system and the MRI device may obtain a magnetic resonance image of the patient at each of the plurality of predetermined positions. Even furthermore, when the patient is positioned at each of a respective one of the predetermined positions, a respective one of a plurality of RF coils installed on a corresponding plurality of body sites of the patient, may be used for obtaining a magnetic resonance image of the patient at that body site.

### BRIEF DESCRIPTION OF THE DRAWINGS

The above and other features and advantages of the present invention will become more apparent by describing in detail exemplary embodiments thereof with reference to the attached drawings in which:
FIG. 1 is a view illustrating a structure of a MRI system according to an embodiment of the present invention;
FIG. 2 is a view illustrating a table device of the MRI system of FIG. 1, according to an embodiment of the present invention;
FIG. 3A is a view illustrating details of a profile acquisition device of the MRI system of FIG. 1, according to an embodiment of the present invention;
FIG. 3B is a view illustrating a profile acquisition device of the MRI system of FIG. 1, according to another embodiment of the present invention;
FIG. 4 is a view illustrating installation of a plurality of RF coils, according to an embodiment of the present invention; and
FIG. 5 is a view illustrating installation of a plurality of RF coils, according to another embodiment of the present invention.
FIG. 6 is a flowchart illustrating a method of controlling an MRI system, according to an embodiment of the present invention.

### DETAILED DESCRIPTION OF_EXEMPLARY EMBODIMENTS

Exemplary embodiments of the present invention will now be described in detail with reference to the accompanying drawings.

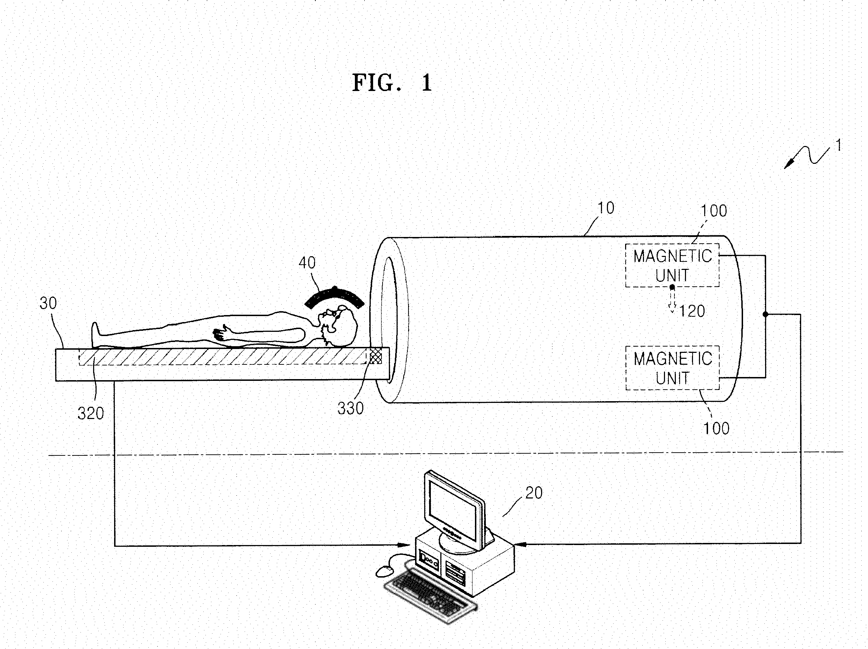

FIG. 1 is a diagram illustrating a structure of a MRI system 1 according to an embodiment of the present invention. Referring to FIG. 1, the MRI system 1 includes a MRI device 10, a control device 20, a table device 30, a radio frequency (RF) coil 40, and a profile acquisition device 320.

In FIG. 1, only constitutive elements of the MRI system 1 are illustrated. Thus, it is well understood by one of ordinary skill in the art that the MRI system 1 may further include other commonly used elements. Thus, the constitutive elements of the MRI system 1 will now be described in more detail and a detailed description of general elements is not provided herein.

The MRI system 1 is a device for non-invasively obtaining magnetic resonance images including information on biological tissues of a patient.

The human body is mostly made up of water. Thus, when a strong magnetic field is applied to the body of a subject, an abnormal cell exhibits a difference in time taken until hydrogen atoms in the body absorb energy with a certain wavelength by a resonance phenomenon and release the energy, as compared to a normal cell. Therefore, the MRI system 1 obtains and analyzes information on cells exhibiting such difference, thereby obtaining magnetic resonance images of desired sites of the body.

That is, the MRI system 1 may be used as a system for obtaining a medical image for diagnosis, such as a magnetic resonance image of a patient, by using a magnetic filed generated by a magnetic force.

The MRI device 10 obtains a magnetic resonance image signal of a body site of a patient by interaction between a main magnetic field generated by a main magnet of a magnetic unit 100 and a gradient magnetic field generated by a gradient coil (not separately shown in unit 100) and the RF coil 40 installed on the body of a patient who lies on the table device 30. The RF coil 40 transmits the magnetic resonance image signal to the control device 20. A general structure of the MRI device 10 and a process of obtaining a magnetic resonance image in the MRI device 10 are obvious to one of ordinary skill in the art and thus a detailed description thereof is not provided herein.

The control device 20 controls the MRI device 10 and the table device 30. Thus, a user controls overall functions and operations of the MRI system 1 by using the control device 20.

In addition, the control unit 20 may display the obtained magnetic resonance image from the MRI device 10. Thus, a user may diagnose a patient by using the displayed magnetic resonance image by the control device 20.

The control device 20 corresponds to a computing device or a user consol of a commonly used MRI system. General structure and functions thereof are obvious to one of ordinary skill in the art and thus a detailed description thereof is not provided herein.

The table device 30 provides a space on which a patient lies to obtain a magnetic resonance image. Thus, the MRI system 1 obtains a magnetic resonance image by transferring a patient who has lain on the table device 30 to a predetermined position inside a magnet of the MRI device 10 and using inside the magnet a magnetic field of the magnetic unit 100 and a magnetic field of the RF coil 40.

In a conventional MRI device, a first laser is installed at an entrance of a magnet and a second laser is installed at a center area of a magnetic unit inside the magnet. When an RF coil is installed on a table device and a center of the RF coil is manually positioned by a radiation therapist at a position of the first laser, the table device can be automatically transferred from a position defined by the first laser to a position defined by the second laser.

Conventionally, if magnetic resonance images of several body sites of a patient, for example, a head and legs need to be sequentially obtained, the following operations are sequentially performed to transfer the table device inside or outside of the magnet in order to obtain an image at each of the body sites.
1. A radiation therapist has a patient lie on the table device and a first RF coil is then installed on a head of the patient.
2. The table device is manually positioned by the radiation therapist at a position of the first laser. Subsequently, the table device is automatically positioned at a position of the second laser and the MRI device obtains a magnetic resonance image of the head of the patient.
3. After the magnetic resonance image of the head is obtained, the table device is put outside of the magnet by the radiation therapist.
4. The first RF coil is removed from the head of the patient by the radiation therapist and a second RF coil is then installed on legs of the patient.
5. The operations 2 to 4 are repeatedly performed to obtain magnetic resonance images for other body sites of the patient.

Thus, traditionally, when magnetic resonance images of several body sites of a patient need to be obtained, RF coils are installed on each of the body sites of the patient, one at a time, by a user of an MRI system (such as a radiation therapist or a doctor), and the table device is manually transferred to the position of the first laser each time when the magnetic resonance image of each body site is to be obtained. Thus, a conventional MRI system takes a long period of time to obtain magnetic resonance images of a plurality of body sites for a patient and users such as radiation therapists or doctors experience not only inconvenience but also a loss of efficiency and therefor also an increase in operating cost.

Furthermore, when magnetic resonance images are traditionally obtained, to adjust a parameter of a pulse sequence of an RF coil based on a specific absorption rate (SAR), the following steps are generally conducted by a user: ask patients for their body weight, or input their body weight arbitrarily, or use an external body weight measuring device. However, an SAR limit varies depending on each patient and thus it is necessary to input accurate body weight information to the MRI system.

To address these problems of the prior art, in accordance with one embodiment of the invention, the MRI system 1 obtains a body profile of a patient and uses the body profile for transferring the position of the patient and adjusting a parameter of a pulse sequence of the RF coil. Hereinafter, structure and functions of the MRI system 1 will be described in more detail.

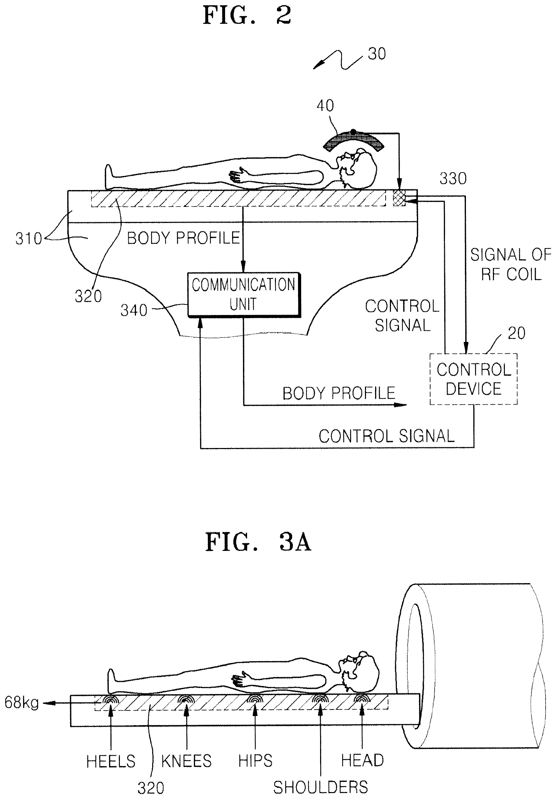

FIG. 2 is a view of a table device 30 of the MRI system 1 of FIG. 1, according to an embodiment of the present invention. In FIG. 2, only constitutive elements of the table device 30 are illustrated. However, it is well understood by one of ordinary skill in the art that the MRI system 1 may further include other commonly used elements.

Referring to FIG. 2, the table device 30 includes a patient-supporting unit 310, a profile acquisition device 320, an RF coil connection unit 330, and a communication unit 340. In FIG. 2, it is illustrated that the table device 30 includes the profile acquisition device 320 and the RF coil connection unit 330, but it is not limited to the above example. For example, the profile acquisition device 320 and the RF coil connection unit 330 may be included in an external device or independently installed.

The profile acquisition device 320 obtains a body profile of a patient. In this regard, the obtained body profile includes body weight information and position information on body sites of the patient. The profile acquisition device 320 may obtain the body profile of a patient while the patient is lying down on the table device 30 outside a magnet of the MRI system 1.

The table device 30 transfers a patient to a predetermined position inside the magnet of the MRI system 1 based on the body profile obtained by the profile acquisition device 320. In particular, the table device 30 transfers a patient by using position information on body sites of the patient among the obtained body profiles such that an RF coil installed on a body site of the patient for obtaining an magnetic resonance image is positioned at a predetermined position.

In this regard, the predetermined position indicates a center position of the magnetic unit 100 of the magnet (corresponding to a position of the second laser 120 of FIG. 1), but is not limited to the above example. For example, the predetermined position may be a position defined by a first laser (not shown) installed at an entrance of the magnet.

FIG. 3A is a view which illustrates details of a profile acquisition device 320 of the MRI system 1 of FIG. 1, according to an embodiment of the present invention. Referring to FIG. 3A, the profile acquisition device 320 obtains position information and body weight information by using at least one pressure sensor installed in the patient-supporting unit 310.

The pressure sensor may be one selected from the group consisting of a piezoresistive pressure sensor, a capacitive pressure sensor, an electromagnetic pressure sensor, and a piezoelectric pressure sensor. However, the pressure sensor is not limited to the above examples and any pressure sensor may be used.

The profile acquisition device 320 obtains body weight information when a patient is lying down by using the pressure sensor. For example, the pressure sensor obtains a body profile of a patient, e.g., a body weight of 68 kg.

In the MRI system 1, in accordance with one embodiment of the invention, the body weight information is used to adjust a parameter of a pulse sequence of the RF coil according to a SAR of an RF coil. In this regard, the parameter of the pulse sequence of the RF coil corresponds to a parameter exhibiting an amplitude of a pulse, a duration of a pulse, or the like. A pulse power or an intensity of a magnetic field is adjusted according to the parameter of the pulse sequence. For example, the pulse power of the RF coil with respect to a heavy weight patient is adjusted by the SAR to be higher than that with respect to a low weight patient.

The MRI device 10 obtains in the magnet room a magnetic resonance image of a patient by using a magnetic field of the RF coil, generated by the parameter of the pulse sequence based on body weight information. Since the body profile exhibiting body weight information is automatically obtained in the profile acquisition device 320, the MRI system does not need to separately adjust the parameter of the pulse sequence of the RF coil by asking a patient for body weight, or arbitrarily set a patient's body weight, or by using an external body weight measuring device.

The profile acquisition device 320 obtains position information of a body site of a patient automatically by using the pressure sensor while a patient is in a lying down position on the table device 30. For example, the profile acquisition device 320 obtains profiles of body sites of a patient, such as a head, a shoulder, hips, knees, heels, and the like that are sensed by the pressure sensor with the patient lying down. However, position information of obtainable body sites is not limited to the above examples.

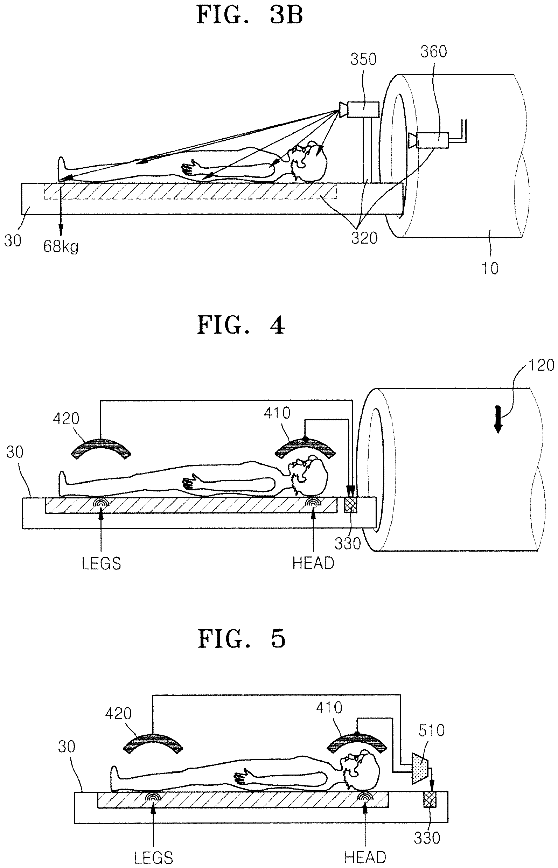

FIG. 3B is a view of a profile acquisition device 320 of the MRI system 1 of FIG. 1, according to another embodiment of the present invention. Referring to FIG. 3B, the profile acquisition device 320 obtains body weight information by using at least one pressure sensor installed in the patient-supporting unit 310 and also obtains position information of a body site of a patient by using a camera 350 installed on the patient-supporting unit 310 or a camera 360 installed in the MRI device 10. In this regard, the position of the camera 350 or 360 is not limited to a certain position. For example, the camera 350 or 360 may be installed at a position other than the table device 30 and the MRI device 10.

A detailed description of the pressure sensor is already provided in the description with regards to FIG. 3A. Unlike the pressure sensor of FIG. 3A, however, the pressure sensor of FIG. 3B obtains only body weight information when a patient is lying down. For example, the pressure sensor obtains a body profile of a patient, e.g., a body weight of 68 kg and may not obtain position information of the body site thereof.

The profile acquisition device 320 obtains position information of a body site of a patient by capturing such information by using the camera 350 or 360 while a patient is lying down. For example, the profile acquisition device 320 obtains profiles of body sites of a patient, such as a head, a shoulder, hips, knees, heels, and the like that are captured using the camera 350 or 360 while the patient is lying down. However, position information of obtainable body sites is not limited to the above examples.

Alternatively, the camera 350 or 360 of the profile acquisition device 320 photographs only the body of a patient and the body profile for position information of each of the body sites may be additionally input by a user via the control device 20.

Referring back to FIG. 2, the table device 30 includes the RF coil 40. The RF coil 40 is installed at a position of a patient's desired body site for obtaining a magnetic resonance image with the patient lying down. The functions of the RF coil 40 installed on the table device 30 are obvious to one of ordinary skill in the art and thus a detailed description thereof is not provided herein.

The RF coil connection unit 330 is connected to at least one RF coil 40 that is installed on at least one body site of a patient. In particular, the RF coil connection unit 330 is wired or wirelessly communicates with the control device 20 to receive a control signal of the control device 20 with respect to a parameter of a pulse sequence of the RF coil 40. In addition, the RF coil connection unit 330 is wired or wirelessly communicates with the RF coil 40 to transmit the control signal of the control device 20 with respect to the parameter of the pulse sequence of the RF coil 40 to the RF coil 40. Also, the RF coil connection unit 330 receives a signal of the RF coil 40 to transmit the signal to the control device 20.

Referring to drawings including FIG. 2, the RF coil connection unit 330 is installed in the patient-supporting unit 310 of the table device 30, but is not limited to the above example. For example, the RF coil connection unit 330 may be included in other devices such as the MRI device 10 and the like or may be independently installed.

The RF coil connection unit 330 may communicate with the control device 20 via the communication unit 340 or may directly communicate with the control device 20.

The RF coil connection unit 330 controls the parameter of the pulse sequence of the RF coil 40. In this regard, the RF coil connection unit 330 controls a parameter of a pulse sequence of the RF coil 40 connected thereto based on body weight information included in the obtained body profile. The RF coil connection unit 330 may control the parameter of the pulse sequence of the RF coil 40 by the control signal of the control device 20.

The patient-supporting unit 310 supports a patient for transferring the patient inside or outside the magnet of the MRI device 10 based on the obtained body profile of the patient when the RF coil 40 is connected to the patient-supporting unit 310. The general structure and operation of the patient-supporting unit 310, for example, a structure of a transfer member, and the like are obvious to one of ordinary skill in the art and thus a detailed description thereof is not provided herein. The patient-supporting unit 310 may be referred to in the art as a patient bed, a patient table, or the like of the MRI system 1.

The communication unit 340 communicates with the control device 20 to transmit the obtained body profile to the control device 20 of the MRI system 1. In addition, the communication unit 340 transmits the signal of the RF coil 40 to the control unit 20. Thus, the communication unit 340 communicates with devices of the MRI system 1 other than the table device 30.

In addition, the communication unit 340 communicates with the control device 20 to receive the control signal of the control device 20. Thus, the communication unit 340 may receive a control signal with respect to a desired body site for obtaining a magnetic resonance image of the obtained body profile. When the control signal is received by the communication unit 340, the table device 30 controls the patient-supporting unit 310 to transfer a position of the RF coil 40 installed on a body site that is exhibited by the control signal to a center position (corresponding to the position of the second laser 120 of FIG. 1) of the magnetic unit 100 inside the magnet. Thus, in this embodiment, there is no necessity for a user such as a radiation therapist to manually transfer the table device 30 to a position of the first laser 110 (not illustrated in drawings), and the table device 30 is automatically transferred to the position of the second laser 120 by the control signal. That is, auto-positioning of a patient is possible, thereby improving the efficiency, and lowering the cost, of the MRI imaging procedure.

As described above, in a conventional MRI system, when magnetic resonance images of several body sites of a patient need to be obtained, an RF coil is manually installed on any one of the body sites of the patient by a user to obtain a magnetic resonance image or that one body site. Thereafter, another RF coil is manually installed on one of the other body sites to obtain a magnetic resonance image of that one body site.

However, the MRI system 1 having improvements in accordance with the present invention may automatically obtain magnetic resonance images of several body sites of a patient without such inconvenient manual operations.

Thus, in accordance with the present invention, when a magnetic resonance image of a single body site of a patient, for example, a head, needs to be obtained, a patient lies down on the patient-supporting unit 310 outside the magnet and the RF coil 40 is installed on the head of the patient by a user as illustrated in FIG. 1 or 2.

Subsequently, the profile acquisition device 320 of the table device 30 obtains a body profile of the patient, e.g., body weight information and position information of body sites. The obtained body profile is transmitted to the control device 20 via the communication unit 340.

The installation position of the RF coil 40 and the position information of body sites included in the body profile are mapped and the mapped information is input by the user to an input unit (not shown) of the control device 20. A storing unit (not shown) of the control device 20 stores the results of the mapped information.

When the user confirms the mapping results of the position of the head of the patient and the RF coil 40 installed thereon via the control device 20, the user inputs to the control device 20 a start command for starting to transfer the table device 30 or starting to obtain a magnetic resonance image.

When the start command is executed in the control device 20, the table device 30 is automatically transferred inside the magnet until the RF coil 40 corresponds to the position of the second laser 120. In this regard, the position of the second laser 120 indicates a center position of the main magnetic field. Subsequently, the MRI device 10 obtains a magnetic resonance image of the head of the patient.

As described above, in the MRI system 1, there is no necessity to manually transfer the table device. Thus, until a process of obtaining a magnetic resonance image of a patient is terminated, the table device 30 is automatically transferred based on the body profile obtained by the table device 30. Thus, a user of the MRI system 1 does not need to experience such inconveniences in the transfer of a traditional table device.

Furthermore, the above described automatic transfer of th patient can be used even when a plurality of RF coils 40 are installed on several body sites of a patient. In this case, the table device 30 is automatically transferred based on the body profile obtained by the table device 30, as described below.

FIG. 4 is a view illustrating a case where a plurality of RF coils are simultaneously installed, according to an embodiment of the present invention.

Thus, even when a plurality of the RF coils 40 are simultaneously installed on several body sites of a patient, the table device 30 is automatically transferred. Accordingly, in the MRI system 1, auto-positioning of a patient with respect to each of the body sites is possible. Referring to FIG. 4, a first RF coil 410 is installed on (or over) a head of a patient, and a second RF coil is installed on (or over) legs of the patient. The first RF coil 410 and the second RF coil 420 are connected to the RF coil connection unit 330.

The profile acquisition device 320 obtains position information of several body sites of a patient. A user maps the positions of the first and second RF coils 410 and 420 and position information of body sites (head, legs) of the patient included in the obtained body profile and inputs the mapped information to an input unit (not shown) of the control device 20. When a start command is executed in the control device 20, the table device 30 is automatically transferred inside the magnet until the position of the first RF coil 410 corresponds to the position of the second laser 120. Next, the MRI device 10 obtains a magnetic resonance image of the head of the patient.

After the acquisition of the magnetic resonance image by the first RF coil 410 is terminated, the second RF coil 420 on the table device 30 is automatically transferred until it corresponds to the position of the second laser 120. Next, the MRI device 10 obtains a magnetic resonance image of the legs of the patient.

As described above, the table device 30 does not need to be manually transferred by a user and is automatically transferred sequentially with respect to body sites of a patient for obtaining magnetic resonance images. Therefore, there is no necessity for a user to manually transfer the table device 30 with respect to each of the body sites of a patient.

FIG. 5 is a view illustrating a case where a plurality of RF coils is simultaneously installed, according to another embodiment of the present invention. A detailed description of elements of FIG. 5 that are the same as those of FIG. 4 is not provided herein. Referring to FIG. 5, the RF coil connection unit 330 includes a multiplexing unit 510. As described above, the RF coil connection unit 330 may be included in a device other than the table device 30.

The multiplexing unit 510 performs a switching operation such that, when a plurality of RF coils are connected to the RF coil connection unit 330, only a signal of any one of the connected RF coils is output at a time. The RF coil connection unit 330 transmits the signal output through the multiplexing unit 510 to the control device 20. Thus, the multiplexing unit 510 acts as a multiplexer or a switch with respect to the first and second RF coils 410 and 420.

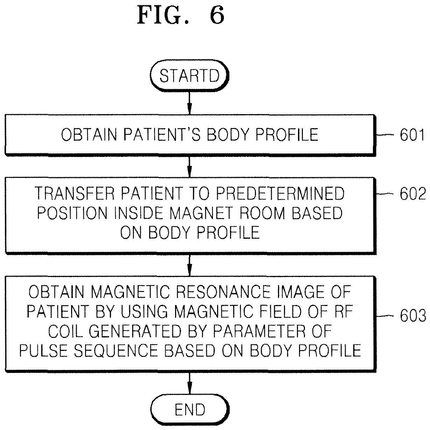

FIG. 6 is a flowchart illustrating a method of controlling an MRI system, according to an embodiment of the present invention. The method of controlling an MRI system includes operations which are time-serially performed by the MRI system 1 illustrated in FIG. 1. Although omitted in the below description, the description of the MRI system 1 of FIG. 1 may be applied to the method of controlling an MRI system.

In accordance with the MRI system (or the method of operating the MRI system) firstly, the profile acquisition device 320 obtains a body profile of a patient (operation 601).

The table device 30 transfers the patient to a predetermined position inside the magnet of the MRI system 1 based on the obtained body profile (operation 602).

The MRI device 10 obtains a magnetic resonance image of the patient by using a magnetic field of the RF coil generated by a parameter of a pulse sequence based on the obtained body profile (operation 603).

As described above, according to the one or more embodiments of the present invention, an MRI system may obtain a body profile such as body weight information or position information of body sites, while the patient is lying down on the table device 30.

Thus, by obtaining body weight information of patients, an effect of an SAR on patients may vary. Therefore, the effect of the SAR on patients may be accurately calculated and an image quality may be improved, accordingly.

In addition, when magnetic resonance images of several body sites of a patient need to be obtained, there is no necessity for a radiation therapist to manually install an RF coil or remove the RF coil and manually transfer a table device, by using such a body profile. Thus, a user such as a radiation therapist may be able to operate the transfer of the table device in a control device of the MRI system, such as a user consol. Thus, auto-positioning of the table device is possible.

Furthermore, the patient turnover rate (that is, MRI efficiency) at hospitals may be increased by use of the auto-positioning provided by the table device of the present invention.

The exemplary embodiments of the present invention described above can be written as computer programs and can be implemented in general-use digital computers , or a special processor or in programmable or dedicated hardware, such as an ASIC or FPGA that execute programs using a computer readable recording medium. In addition, a data structure used in the embodiments of the present invention may be recorded on a computer-readable recording medium in a variety of ways. Examples of the computer-readable recording medium include magnetic storage media (e.g., ROM, floppy disks, hard disks, etc.) and optical recording media (e.g., CD-ROMs, or DVDs). Alternatively, software or computer code can be downloaded over a network and stored, and when such software or computer code is accessed and executed by the computer, processor or hardware, implement the processing methods described herein.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A magnetic resonance imaging (MRI) system comprising:
a profile acquisition device for obtaining a body profile of a patient;
a table device for transferring the patient to a predetermined position inside a magnet of the MRI system based on the obtained body profile; and
an MRI device for obtaining in the magnet a magnetic resonance image of the patient by using a magnetic field of an RF coil generated by a parameter of a pulse sequence based on the obtained body profile.

2. The MRI system of claim 1, wherein the obtained body profile comprises both body weight information of the patient and position information of body sites of the patient.

3. The MRI system of claim 2, wherein the table device is transferred such that an RF coil installed on a body site of a patient for obtaining a magnetic resonance image is positioned at the predetermined position.

4. The MRI system of claim 3, wherein, when a plurality of the RF coils are installed on a plurality of the body sites for obtaining a magnetic resonance image, the table device is transferred such that the plurality of the RF coils installed on the plurality of the body sites are sequentially positioned at the predetermined position.

5. The MRI system of claim 2, wherein the parameter of the pulse sequence is controlled according to a specific absorption rate (SAR) corresponding to the obtained body weight information.

6. The MRI system of claim 1, wherein the profile acquisition device comprises at least one pressure sensor installed in the table device.

7. The MRI system of claim 6, wherein the profile acquisition device obtains the body profile comprising both body weight information of the patient and position information of body sites of the patient, by using the pressure sensor while the patient is lying down on the table device.

8. The MRI system of claim 1, wherein the profile acquisition device comprises at least one pressure sensor installed in the table device and at least one camera installed in the MRI system.

9. The MRI system of claim 8, wherein the profile acquisition device obtains body weight information of the patient among the body profiles by using the pressure sensor and obtains position information of body sites of the patient among the body profiles by using the camera.

10. The MRI system of claim 1, further comprising a control device for controlling a transfer position of the table device and the parameter of the pulse sequence based on the obtained body profile.

11. The MRI system of claim 10, wherein the control device maps and stores position information of body sites included in the obtained body profile and an installation position of the RF coil.

12. The MRI system of claim 10, wherein the table device comprises a communication unit that communicates with the control device to receive a control signal of the control device with respect to a body site of a patient for obtaining a magnetic resonance image and is transferred according to the control signal received by the communication unit.

13. The MRI system of claim 10, further comprising an RF coil connection unit that is wired or wirelessly communicates with the control device to receive a control signal of the control device with respect to the parameter of the pulse sequence, is wired or wirelessly communicates with the RF coil to transmit the control signal of the control device with respect to the parameter of the pulse sequence to the RF coil, and receives a signal of the RF coil to transmit the signal to the control device.

14. The MRI system of claim 13, wherein the RF coil connection unit further comprises a multiplexing unit that performs a switching operation such that, if a plurality of the RF coils are connected to the RF coil connection unit, only a signal of any one of the plurality of the RF coils is output at a given time, and the RF coil connection unit transmits to the control device the signal of any one of the plurality of the RF coils that is output through the multiplexing unit.

15. A method of controlling a magnetic resonance imaging (MRI) system, the method comprising:
obtaining a body profile of a patient;
transferring the patient to a predetermined position inside a magnet of the MRI system based on the obtained body profile; and
obtaining a magnetic resonance image of the patient by using a magnetic field of an RF coil generated by a parameter of a pulse sequence based on the obtained body profile.
